# EUROPÄISCHE PATENTSCHRIFT

(11) **EP 1 259 185 B1**
(45) Veröffentlichungstag und Bekanntmachung des Hinweises auf die Patenterteilung: **29.08.2007**
(21) Anmeldenummer: 01971825.3
(22) Anmeldetag: 31.07.2001
(51) Int. Cl.: A61B 19/00

(54) **VERFAHREN ZUM NAVIGIEREN IM KÖRPERINNEREN ANHAND DREIDIMENSIONAL VISUALISIERTER STRUKTUREN**
METHOD FOR NAVIGATING IN THE INTERIOR OF THE BODY USING THREE-DIMENSIONALLY VISUALISED STRUCTURES
PROCEDE POUR NAVIGUER A L'INTERIEUR DU CORPS AU MOYEN DE STRUCTURES VISUALISEES EN TROIS DIMENSIONS

(30) Priorität: 01.08.2000 DE 10037491
(43) Veröffentlichungstag der Anmeldung: 27.11.2002
(73) Patentinhaber: Stryker Leibinger GmbH & Co. KG, 79111 Freiburg (DE)
(72) Erfinder: SCHWEIKARD, Achim, 20357 Hamburg (DE); DOETTER, Manfred, 81679 München (DE); ROTH, Michael, 86153 Augsburg (DE); MOCTEZUMA de la BARRERA, José-Luis, 79104 Freiburg (DE)
(74) Vertreter: Röthinger, Rainer
(86) Internationale Anmeldenummer: PCT/EP2001/008844
(87) Internationale Veröffentlichungsnummer: WO 2002/009611

## Beschreibung

Die Erfindung betrifft eine rechnergestützt erzeugte Navigationshilfe zum Navigieren beispielsweise eines chirurgischen Instruments oder eines Endoskops im Körperinneren anhand dreidimensional visualisierter Strukturen.

Während chirurgischer Operationen wird in der Regel angestrebt, einen möglichst kleinen Schnitt durch die Haut des Patienten vorzunehmen. Dieser Schnitt ist oftmals gerade groß genug, um die zu behandelnde anatomische Struktur im Körperinneren mit chirurgischen Instrumenten erreichen zu können. Aus diesem Grund ist es für den Chirurgen schwierig und oftmals sogar unmöglich, die operationsrelevanten anatomischen Strukturen des Patienten optisch zu erfassen. Auch die Lage z. B. chirurgischer Instrumente relativ zu einem zu behandelnden anatomischen Objekt ist oftmals für den Chirurgen nicht einsehbar. Aus diesem Grund gewinnen Verfahren zur Visualisierung von Strukturen im Körperinneren zum Zweck der Navigation derartiger Instrumente zunehmend an Bedeutung.

In der DE 198 07 884 A1 wird ein Verfahren zum Navigieren eines chirurgischen Instruments im Körperinneren beschrieben. Gemäß diesem Verfahren werden mittels einer Aufnahmevorrichtung mehrere Aufnahmen aus unterschiedlichen Positionen und Orientierungen gemacht und die relativen Positionen und Orientierungen der Aufnahmen zueinander bestimmt. Anschließend wird mit Hilfe dieser Aufnahmen intraoperativ die relative Position eines anatomischen Zielobjekts zu dem chirurgischen Instrument ermittelt und visualisiert.

Sowohl für die exakte Planung einer Operation als auch für die optische Überwachung des Operationsverlaufes sind zweidimensionale Aufnahmen wie Röntgenaufnahmen in der Regel nicht ausreichend. Es besteht daher das Erfordernis, dreidimensionale Darstellungen einzusetzen.

Derartige dreidimensionale Darstellungen lassen sich beispielsweise mittels der Computertomographie (CT) erzeugen. CT-Verfahren werden aufgrund der apparativen Gegebenheiten und aufgrund des hohen Rechenaufwandes in erster Linie zur Gewinnung präoperativer Daten verwendet. Für eine intraoperative, d.h. während der Operation erfolgende Visualisierung sind CT-Verfahren daher nicht geeignet. Weiterhin ist in vielen Fällen die mit den CT-Verfahren einhergehende hohe Strahlenbelastung für den Patienten unerwünscht.

Zur Vermeidung hoher Strahlenbelastungen kann daran gedacht werden, die zur Planung der Operation und zur Überwachung des Operationsverlaufs erforderliche, dreidimensionale Darstellung aus zwei oder mehr zweidimensionalen Darstellungen zu generieren. Ein derartiges Verfahren wird beispielsweise in der US 4,899,318 beschrieben.

Die US 5,852,646 beschreibt ein Verfahren, bei dem aus einer Serie von Aufnahmen aus verschiedenen Perspektiven eine dreidimensionale Repräsentation des Objekts berechnet wird, die dann auf einer zweidimensionalen Anzeige ausgegeben wird. Eine solche Darstellung bezieht sich nicht mehr unmittelbar auf die verwendeten Aufnahmen, sondern auf das durch Berechnung generierte Objekt oder auf ein extrahiertes geometrisches Attribut (Punkt, Linie, etc.).

Der Erfindung liegt die Aufgabe zugrunde, ein System und ein präoperatives Verfahren zum rechnergestützten Erzeugen eines graphischen Navigationshilfe anhand dreidimensional visualisierter Strukturen im Körperinneren anzugeben, welches hohe Strahlenbelastungen des Patienten vermeidet, mit wenig Rechenaufwand verbunden ist und daher insbesondere auch für den intraoperativen Einsatz geeignet ist.

Erfindungsgemäß wird diese Aufgabe dadurch gelöst, dass in einem ersten Schritt mindestens zwei zweidimensionale Aufnahmen desselben anatomischen Objekts aus unterschiedlichen Aufnahmepositionen sowie Informationen über die jeweilige räumliche Lage des Aufnahmesystems relativ zum anatomischen Objekt bereitgestellt werden. In einem zweiten Schritt wird anschließend in jeder der zweidimensionalen Aufnahmen eine Projektion einer zu visualisierenden geometrischen Struktur festgelegt, wobei die zu visualisierende geometrische Struktur vom anatomischen Objekt verschieden und aus zwei Aufnahmen eindentig ermittelbar ist. In einem dritten Schritt wird für jede der Aufnahmen eine Kegelfläche im Raum generiert, wobei die räumliche Lage von Kegelspitze und Kegelleitkurve aus der räumlichen Lage des Aufnahmesystems und die Form der Kegelleitkurve aus der festgelegten Projektion der zu visualisierenden geometrischen Struktur auf der jeweiligen Aufnahme ermittelt werden. In einem vierten Schritt wird der räumliche Schnitt der einzelnen Kegelflächen gebildet, wobei dieser räumliche Schnitt der im Körperinneren zu visualisierenden geometrischen Struktur entspricht. Anschließend wird die derart ermittelte geometrische Struktur oder ein Schnitt mehrerer derart ermittelter geometrischer Strukturen oder sowohl die Strukturen als auch deren Schnitt graphisch als Navigationshilfe dargestellt.

Zweckmäßigerweise wird als Navigationshilfe zusätzlich zu der geometrischen Navigationsstruktur auch noch entweder das anatomische Objekt oder ein z.B. chirurgisches Instrument, oder beide, dreidimensional graphisch dargestellt. Die dreidimensionale Darstellung des anatomischen Objekts und des chirurgischen Instruments kann dabei auf die gleiche oder auf ähnliche Weise wie die dreidimensionale Darstellung der geometrischen Struktur gewonnen werden.

Gemäß der vorliegenden Erfindung wird folglich aus auf wenigen zweidimensionalen Aufnahmen festgelegten Projektionen ein dreidimensionales Modell der zu visualisierenden geometrischen Struktur erstellt. Da im einfachsten Fall lediglich zwei zweidimensionale Aufnahmen vorhanden sein müssen, und diese beiden Aufnahmen nicht notwendigerweise mit einem bildgebenden Verfahren, welches Röntgenstrahlung oder andere energiereiche Strahlung verwendet, erstellt sein müssen, ist bei dem erfindungsgemäßen Visualisierungsverfahren die Strahlenbelastung für den Patienten im Vergleich zu herkömmlichen CT-Verfahren vernachlässigbar gering. Da außerdem auch die zu bearbeitenden Datenmengen insbesondere im Vergleich zu CT-Verfahren relativ gering sind, kann die Visualisierung äußerst schnell und sogar intraoperativ durchgeführt werden, um dem Chirurgen aktuelle Informationen über den Verlauf der Operation zur Verfügung zu stellen. Weiterhin kann die Strahlenbelastung für das OP-Team erheblich reduziert werden.

Das Festlegen der Projektionen auf den Aufnahmen kann auf unterschiedliche Art und Weise geschehen, beispielsweise automatisch, teilautomatisch oder manuell. Zweckmäßigerweise erfolgt das Festlegen derart, dass nach dem Festlegen Informationen betreffend die festgelegten Projektionen als digitale Daten zur Verfügung stehen und zum Zweck der Visualisierung rechnergestützt weiterverarbeitet werden können.

Beim Festlegen der Projektion der geometrischen Struktur auf den einzelnen Aufnahmen wird gleichzeitig die Gestalt der zu visualisierenden geometrischen Struktur bestimmt. Die in einer bestimmten Aufnahme festzulegende Projektion kann beispielsweise ein Punkt, eine Gerade, ein Kreissegment oder eine beliebige andere linienartige Struktur sein. Bei der zu visualisierenden geometrischen Struktur kann es sich um eine beliebige eindimensionale, zweidimensionale oder dreidimensionale Struktur wie einen Punkt, eine Gerade, eine Ebene oder eine Kugel handeln.

Die zu visualisierende geometrische Struktur stimmt nicht mit dem auf den zweidimensionalen Aufnahme abgebildeten anatomischen Objekt überein, kann aber aus diesem abgeleitet sein. So ist es beispielsweise möglich, auf einer Aufnahme eines Femurknochens eine Kreissegmentprojektion im Bereich des kugelförmigen Kopfes des Femurknochens festzulegen, um eine Navigationshilfe in Gestalt einer geometrischen Kugelstruktur zu visualisieren. Das Navigieren anhand derart visualisierter geometrischer Strukturen hat gegenüber dem Navigieren anhand beispielsweise des Femurknochens oder eines Modells des Femurknochens den Vorteil, dass es einfacher, übersichtlicher und daher auch sicherer und schneller ist. Daher lässt sich mittels eines solchen Navigierens nicht nur die Operationsdauer verkürzen, sondern auch die Operationssicherheit erhöhen.

Die Ermittlung von Informationen, welche einen Rückschluss auf die räumlichen Lagen des Aufnahmesystems gestatten, in der die Aufnahme aufgenommen wurde, kann auf vielfältige Weise geschehen. So kann beispielsweise die räumliche Lage des Aufnahmesystems unveränderlich und, in der Regel, im Voraus bekannt sein, und die Lage des anatomischen Objekts von Aufnahme zu Aufnahme geändert werden. Aus der jeweiligen Lage des anatomischen Objekts lässt sich dann unter der Annahme eines ortsfesten, anatomischen Objekts auf die jeweiligen fiktiven Aufnahmepositionen zurückrechnen.

Andererseits ist es auch möglich und oftmals sogar zweckmäßig, das anatomische Objekt ortsfest im Raum zu platzieren und die räumliche Lage des Aufnahmesystems von Aufnahme zu Aufnahme zu verändern. Zur Ermittlung der jeweiligen räumlichen Lagen des Aufnahmesystems wird in diesem Fall vorzugsweise mit einem aus dem Stand der Technik bekannten Tracking-System gearbeitet. Bei Verwendung eines Röntgen-Aufnahmesystems mit einem C-Bogen kann am C-Bogen ein Marker für ein im Raum installiertes Infrarot-Tracking-System angebracht sein. Die genaue räumliche Lage des C-Bogens, welche einen Rückschluss auf die räumlichen Lagen einer Röntgenquelle und eines Röntgendetektors gestattet, kann dann über das Tracking-System ermittelt werden. Durch die räumliche Lage der Röntgenquelle und des Röntgendetektors ist die räumliche Lage der Kegelfläche bestimmt. Weiterhin kann die Lage des C-Bogens bestimmt werden, indem ein ortsfester Roboter verwendet wird. Der Roboter bewegt ein mit röntgendichten Marken versehenes Kalibrierobjekt in den Strahlengang des C-Bogens. Aus der Lage der Marken im Bild kann die Stellung des C-Bogens berechnet werden.

Die einzelnen zweidimensionalen Aufnahmen können auf unterschiedliche Art erstellt werden. Je nach Aufnahmemethode werden die räumlichen Lagen von Kegelspitze und Kegelleitkurve auf unterschiedliche Weise bestimmt. Bei dem bereits erwähnten, bevorzugt zum Einsatz kommenden Röntgen-Aufnahmeverfahren ist das anatomische Objekt, und gegebenenfalls auch das chirurgische Instrument, zwischen einer Röntgenquelle und einem Röntgendetektor angeordnet. Im einfachsten Fall entspricht die räumliche Lage der Röntgenquelle der räumlichen Lage der Kegelspitze und die räumliche Lage des Röntgendetektors der räumlichen Lage der Kegelleitkurve. Sofern Röntgenoptiken verwendet werden, lässt sich aus den Kenndaten der Röntgenoptiken auf die räumlichen Lagen von Kegelspitze und Kegelleitkurve zurückrechnen. Auch wenn die zweidimensionalen Aufnahmen durch andere bildgebende Verfahren als Röntgenverfahren (Infrarot-Verfahren, Ultraschall-Verfahren, MR-Verfahren, usw.) erstellt wurden, ist es in der Regel erforderlich, zusätzlich die jeweiligen Systemparametern des Aufnahmesystems bei der Ermittlung der räumlichen Lagen von Kegelspitze und Kegelleitkurve zu berücksichtigen.

Vorteilhafterweise wird für jede zweidimensionale Aufnahme eine Kegelfläche im Raum generiert. Eine Kegelfläche entsteht durch Bewegen einer Geraden, welche durch einen festen Punkt, die Kegelspitze, geht, längs einer Kurve, der Kegelleitkurve. Die Kegelleitkurve bestimmt folglich die Form der Kegelfläche.

Die Kegelleitkurve kann eine Gerade sein, welche etwa als vom Chirurgen angestrebte Vorzugsrichtung in den einzelnen Aufnahmen festgelegt ist. Die Vorzugsrichtung kann diejenige Richtung kennzeichnen, aus welcher sich z.B. ein Endoskop oder ein chirurgisches Instrument einem zu behandelnden, anatomischen Objekt nähern soll. Für den Fall, dass die Kegelleitkurve eine Gerade ist, weist die zugehörige Kegelfläche eine Dreiecksgestalt auf.

Im Extremfall kann die Kegelleitkurve auch ein Punkt sein, welcher beispielsweise als Zielposition in jeder der Aufnahmen festgelegt ist. Diese Zielposition kann eine Stelle kennzeichnen, an welcher ein chirurgischer Eingriff stattfinden soll. Bei einer punktförmigen Kegelleitkurve weist die zugehörige Kegelfläche die Gestalt einer Geraden auf.

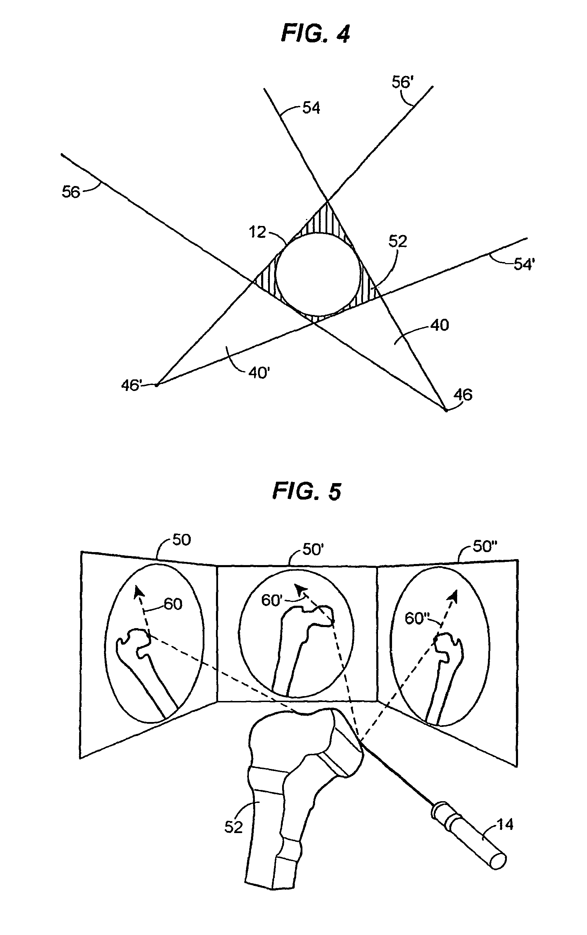

Im Gegensatz zur dreidimensionalen Visualisierung eines anatomischen Objekts wie eines Femurknochens, welche anhand lediglich zweier Aufnahme nur näherungsweise erfolgen kann (s. Fig. 4), können eine zu visualisierende Vorzugsrichtung oder eine zu visualisierende Kugel mittels zweier Aufnahmen, d.h. mittels des Schnitts zweier Kegel, exakt und eindeutig festgelegt werden.

Gemäß einer bevorzugten Ausführungsform der Erfindung, welche auch unabhängig von der Visualisierung geometrischer Strukturen zum Einsatz gelangen kann, wird nach dem Bilden des räumlichen Schnittes einzelner Kegelflächen mindestens ein weiterer Datensatz, welcher präoperativ oder intraoperativ erstellt wurde, zur verfeinernden Korrektur z.B. eines Näherungsmodells eines anatomischen Objekts herangezogen. Bei diesem weiteren Datensatz kann es sich um einen Datensatz handeln, welcher durch ein Magnetoresonanz-Verfahren (MR-Verfahren) erstellt wurde. Die MR-Tomographie besitzt den Vorteil, dass sie mit keiner Strahlenbelastung für den Patienten verbunden ist. Es kann beispielsweise eine dreidimensionale Darstellung eines anatomischen Objekts, welche aus zweidimensionalen Röntgenaufnahmen generiert wurde, mittels einer oder mehrerer MR-Aufnahmen verfeinert werden. Umgekehrt ist es möglich, eine dreidimensionale Darstellung des anatomischen Objekts, welche aus zweidimensionalen MR-Aufnahmen gewonnen wurde, mit Hilfe zusätzlich aufgenommener Röntgenbilder zu verfeinern.

So kann beispielsweise daran gedacht werden, in einem ersten Schritt mindestens zwei zweidimensionale Röntgen-Aufnahmen desselben Knochens aus unterschiedlichen Aufnahmepositionen, Informationen, welche einen Rückschluss auf die jeweilige räumliche Lage des Röntgen-Aufnahmesystems relativ zum Knochen gestatten, sowie einen MR-Datensatz des Knochens bereitzustellen. Anschließend kann eine Projektion einer Oberfläche oder eines Umrisses der Spongiosa des Knochens in jeder zweidimensionalen Röntgen-Aufnahme festgelegt werden und für jede Röntgen-Aufnahme eine Kegelfläche im Raum generiert werden, wobei die räumlichen Lagen von Kegelspitze und Kegelleitkurve aus der jeweiligen räumlichen Lage des Aufnahmesystems und die Form der Kegelleitkurve aus der Form der Projektion ermittelt werden. Zur Ermittlung eines ersten Modells der Oberfläche der Spongiosa kann ein räumlicher Schnitt der einzelnen Kegelflächen gebildet werden und aus dem MR-Datensatz kann ein zweites Modell der Spongiosa ermittelt werden. Eine Darstellung des Knochens lässt sich dann durch Kombination der beiden Modelle erzeugen, wobei anhand der Darstellung z.B. navigiert werden kann. Das erste dreidimensionale Modell kann auch auf anderer Weise als vorstehend erläutert ermittelt werden.

Eine Korrektur der aus dem räumlichen Schnitt der Kegelflächen resultierenden geometrischen Struktur oder eines Näherungsmodells des anatomischen Objekts kann auch durch einen solchen Datensatz erfolgen, welcher ein generisches, formvariables Modell der Struktur oder des Objekts enthält. Ein derartiges, generisches Modell kann beispielsweise aus anatomischen Atlanten abgeleitet werden oder aus einem exakten Modell eines nach Alter, Geschlecht, Herkunft usw. vergleichbaren Patienten gewonnen werden.

Gemäß einem weiteren Aspekt der Erfindung wird nach der Berechnung einer dreidimensionalen Darstellung z.B. einer geometrischen Struktur, eines anatomischen Objekts oder eines chirurgischen Instruments eine inverse Berechnung angestellt, um geeignete räumliche Lagen des Aufnahmesystems für weitere zweidimensionale Aufnahmen zur Verbesserung der Darstellung zu ermitteln. So können dem Chirurgen Aufnahmerichtungen für den C-Bogen eines Röntgen-Aufnahmesystems angezeigt werden, aus denen eine Durchleuchtung aufgrund der bisherigen Berechnungen sinnvoll erscheint.

Außer der zu behandelnden, anatomischen Struktur kann gleichzeitig die aktuelle Position eines zu navigierenden Instrumentes wie eines chirurgischen Instrumentes, eines Endoskops usw. dargestellt werden. Die Position des Instrumentes relativ zur zu behandelnden, anatomischen Struktur kann auf unterschiedliche Art und Weise ermittelt werden. So kann beispielsweise am Instrument der Marker eines Tracking-Systems angebracht werden und die aktuelle Position des Instrumentes mit Hilfe des Tracking-Systems ermittelt werden. Andererseits wäre es auch denkbar, die Position des Instruments mit Hilfe des erfindungsgemäßen Visualisierungsverfahren zu ermitteln. Vorzugsweise wird außer dem Instrument auch noch die Wirkachse des Instruments dargestellt. Die Wirkachse gibt z.B. diejenige Richtung an, in welcher ein chirurgisches Instrument zur Anwendung kommt. Weiterhin kann noch der aktuelle Abstand des Instruments von dem zu behandelnden, anatomischen Objekt angezeigt werden.

Außer der zu visualisierenden geometrischen Struktur können gleichzeitig auch noch die einzelnen zweidimensionalen Aufnahmen, welche der Visualisierung der geometrischen Struktur zugrundeliegen, graphisch dargestellt werden. Die einzelnen zweidimensionalen Aufnahmen können in der Darstellung derart angeordnet werden, dass ihre räumliche Lage in der graphischen Darstellung mit ihrer jeweiligen Aufnahmeposition korrespondiert.

Gemäß einer weiteren Ausführungsform der Erfindung kann in der vorstehend beschriebenen graphischen Darstellung der zu visualisierenden geometrischen Struktur oder in einer separaten Darstellung eine Navigationshilfe für das Instrument abgebildet sein. Die Navigationshilfe zeigt z.B. die aktuelle Position der Wirkachse des chirurgischen Instruments relativ zu einer visualisierenden Vorzugsrichtung in Gestalt einer Tunnelstruktur an. Die Navigationshilfe kann weiterhin Richtungspfeile umfassen, welche das Ausrichten der Wirkachse entlang der Vorzugsrichtung erleichtern.

Weitere Vorteile und Einzelheiten der Erfindung ergeben sich aus den Ausführungsbeispielen und den Figuren. Es zeigt:
- Fig. 1: eine schematische Darstellung eines operativen Eingriffs unter Zuhilfenahme eines Tracking-Systems;
- Fig. 2: eine schematische Darstellung einer Kegelfläche;
- Fig. 3: die Ermittlung eines Modells einer zu visualisierenden Struktur unter Zuhilfenahme des räumlichen Schnitts zweier Kegelflächen;
- Fig. 4: die Navigation eines chirurgischen Instruments unter Zuhilfenahme der zweidimensionalen Aufnahmen und des Modells der zu visualisierenden Struktur;
- Fig. 5: eine zweidimensionale Schnittansicht des räumlichen Schnitts zweier Kegelflächen;
- Fig. 6: eine erfindungsgemäße Darstellung eines chirurgischen Eingriffs auf einem Bildschirm;
- Fig. 7: einen geplanten operativen Eingriff mit einem chirurgischen Bohrer;
- Fig. 8: zwei Röntgenaufnahmen zur Vorbereitung des chirurgischen Eingriffs gemäß Fig. 7;
- Fig. 9: die Ermittlung eines Modells der gemäß Fig. 7 zu visualisierenden Struktur; und
- Fig. 10 bis 12: eine erfindungsgemäße Navigationshilfe.

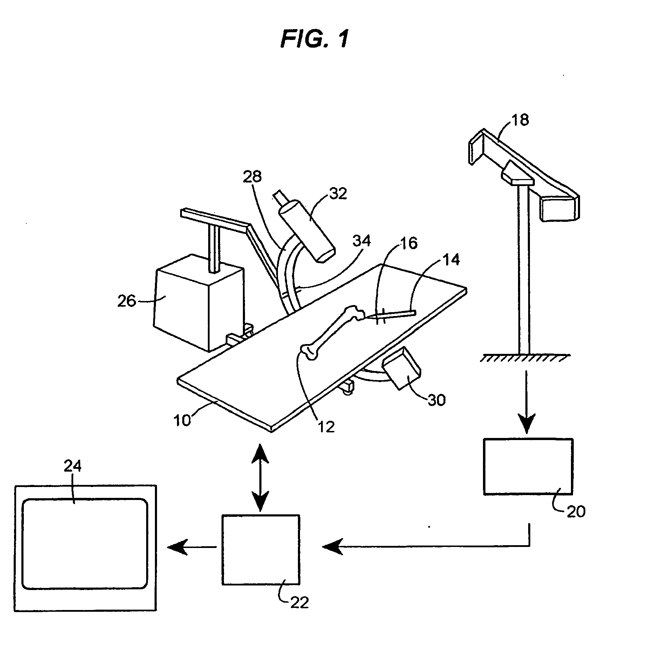

In Fig. 1 ist ein Operationsszenario auf der Grundlage des erfindungsgemäßen präoperativen Verfahrens schematisch dargestellt. Auf einem Operationstisch 10 ist ein operativ zu behandelndes anatomisches Objekt in Gestalt eines Femurknochens 12 angeordnet. Der operative Eingriff wird unter Zuhilfenahme eines chirurgischen Instruments 14 durchgeführt. An dem chirurgischen Instrument 14 ist ein Marker 16 eines Infrarot-Tracking-Systems befestigt. Dieser Marker 16 ist zum Senden von infraroter Strahlung ausgebildet. Das Infrarot-Tracking-System umfasst weiterhin einen ortsfest angeordneten Infrarotdetektor 18. Ein Lokalisierungsrechner 20 des Tracking-Systems errechnet aus den vom Infrarot-Detektor 12 empfangenen Signalen die aktuelle räumliche Lage des mit dem Marker 16 versehenen chirurgischen Instruments 14. Die errechneten räumlichen Koordinaten werden vom Lokalisierungsrechner 20 über einen Datenbus an einen Zentralrechner 22 übermittelt, dort graphisch aufbereitet und auf einem Bildschirm 24 graphisch dargestellt.

In Fig. 1 ist weiterhin ein Röntgen-Aufnahmesystem 26 mit einem C-Bogen 28 dargestellt. An einem der beiden Enden des C-Bogens 28 ist eine Röntgenquelle 30 und an dem gegenüberliegenden Ende des C-Bogens 28 ein Röntgendetektor 32 angeordnet. Die von dem Röntgen-Aufnahmesystem 26 erstellten, zweidimensionalen Aufnahmen werden über einen Datenbus dem Zentralrechner 22 in digitalisierter Form zugeführt. Gleichzeitig dient der Zentralrechner 22 auch zur Steuerung des Röntgen-Aufnahmesystems 26.

Bereits im Vorfeld des operativen Eingriffs wurden durch das Röntgen-Aufnahmesystem 26 eine Mehrzahl zweidimensionaler Aufnahmen erstellt und im Zentralrechner 22 erfindungsgemäß verarbeitet. Beim Röntgen-Aufnahmesystem 26 ist die räumliche Lage der Aufnahmeposition durch die räumliche Lage der Röntgenquelle 30 und die räumliche Lage der Bildebene durch die räumliche Lage des Röntgendetektors 32 vorgegeben. Zur Bestimmung der räumlichen Lage von Röntgenquelle 30 und Röntgendetektor 32 ist am C-Bogen 28 des Röntgen-Aufnahmesystems 26 ein weiterer Marker 34 befestigt, dessen Aufbau mit dem Aufbau des am chirurgischen Instrument 14 angeordneten Markers 16 übereinstimmt. Mittels des Infrarotdetektors 18 des Infrarot-Tracking-Systems kann somit neben der räumlichen Lage des chirurgischen Instruments 14 auch die räumliche Lage der Aufnahmeposition und der Bildebene ermittelt werden. Die diesbezüglichen Informationen werden ebenfalls über den Lokalisierungsrechner 20 dem Zentralrechner 22 zur Verfügung gestellt.

Der Zentralrechner 22 generiert aus den präoperativ gewonnenen zweidimensionalen Aufnahmen ein dreidimensionales Näherungsmodell des Femurknochens 12 und stellt dieses Näherungsmodell, eine zu visualisierende geometrische Struktur sowie die relative Lage des chirurgischen Instruments 14 zum Femurknochen 12 bzw. zur geometrischen Struktur auf dem Bildschirm 24 graphisch dar.

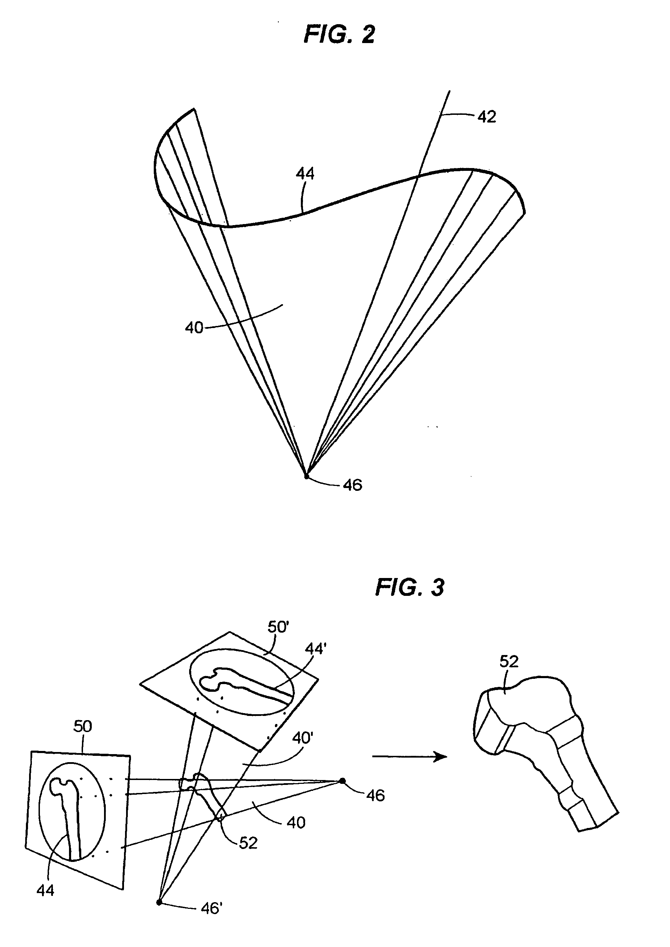

Nachfolgend wird unter Bezugnahme auf die Fig. 2 bis 12 die Durchführung des erfindungsgemäßen Visualisierungsverfahrens näher erläutert.

In Fig. 2 ist eine Kegelfläche 40 dargestellt. Diese Kegelfläche 40 entsteht durch Bewegung einer Geraden 42, welche durch die Kegelspitze 46 verläuft, längs der Kegelleitkurve 44. Die Kegelfläche erstreckt sich ausgehend von der Kegelspitze 46 entlang der Geraden 42 ins Unendliche und schließt die Leitkurve 44 mit ein.

Nachdem in Fig. 2 das Generieren einer Kegelfläche beispielhaft erläutert wurde, erschließt sich die dreidimensionale Visualisierung mittels Kegelflächen durch Betrachten von Fig. 3. In Fig. 3 sind zwei Kegelflächen 40 und 40' in ihrer relativen räumlichen Anordnung zueinander dargestellt. Weiterhin dargestellt in Fig. 3 sind zwei zweidimensionale Aufnahmen 50, 50', welche von dem in Fig. 1 dargestellten Röntgen-Aufnahmesystem 26 aufgenommen wurden. Die räumlichen Lagen der Kegelspitzen 46, 46' der beiden Kegel 40, 40' sind durch die räumlichen Lagen der Röntgenquelle zum jeweiligen Aufnahmezeitpunkt der beiden Aufnahmen 50, 50' festgelegt. Die Form jeder Kegelleitkurve 44, 44' ist durch den jeweiligen Umriss des in den Aufnahmen 50, 50' beispielhaft dargestellten Femurknochens bestimmt. In Fig. 3 sind die beiden Aufnahmen 50, 50' in derjenigen räumlichen Lage angeordnet, in welcher sich die jeweilige Bildebene befand.

Durch Bilden des räumlichen Schnitts der Kegelfläche 40 mit der Kegelfläche 40' erhält man ein Näherungsmodell 52 des anatomischen Objekts in Gestalt des in Fig. 1 im Original dargestellten Femurknochens 12.

Wie Fig. 3 entnommen werden kann, ist das Näherungsmodell 52 des Femurknochens noch vergleichsweise grob. Eine Verfeinerung des Modells 52 kann dadurch erfolgen, dass neben den beiden Aufnahmen 50, 50' noch weitere Aufnahmen des Femurknochens 12 aus zusätzlichen Aufnahmepositionen aufgenommen werden und das Näherungsmodell nachfolgend auf der Grundlage des räumlichen Schnitts von drei oder mehr Kegelflächen ermittelt wird.

Eine weitere Möglichkeit, um das Näherungsmodell 52 zu verbessern, besteht darin, bei der Erstellung des Näherungsmodells 52 einen präoperativ ermittelten MR-Datensatz des in Fig. 1 dargestellten Femurknochens zu berücksichtigen. Auch mit Hilfe der MR-Tomographie lässt sich nämlich ein dreidimensionales Modell des Femurknochens 12 berechnen.

Ein typischer Knochen ist aufgebaut aus einem weichen Kerngewebe (Spongiosa), der umhüllt ist von einer dünnen Schicht aus härterem Gewebe (Kortikalis). Die Dicke der Kortikalis-Schicht ist variabel. Obwohl der Einsatz von MR-Tomographie wünschenswert ist, ist in MR-Bildern die Kortikalis-Schicht sehr schwer automatisch zu segmentieren. Allerdings ist sowohl der Rand der Kortikalisschicht, als auch der darunterliegende Rand der Spongiosa im Röntgenbild sichtbar. Ein grundsätzliches Problem bei der MR-Tomographie besteht also darin, dass lediglich die Spongiosa des Femurknochens gut sichtbar abgebildet wird, während die äußere Kortikalisschicht schwarz erscheint. Das aus dem MR-Datensatz erhältliche dreidimensionale Modell stimmt somit nicht mit der tatsächlichen Gestalt des Femurknochens 12 überein, da die äußere Kortikalisschicht fehlt. Trotzdem erlaubt das MR-Modell Rückschlüsse auf die Form des Femurknochens, welche zur Verfeinerung des in Fig. 3 dargestellten Näherungsmodells 52 verwendet werden können.

Daher kann folgendermaßen vorgegangen werden: Der Rand der Spongiosa wird in den Röntgenaufnahmen markiert, und es wird die Oberfläche der Spongiosa im Kernspinbild automatisch segmentiert. Anschließend kann zum Registrierungsschritt (räumlicher Abgleich der Lage von Kernspinbild zu Röntgenbildern) ein Verfahren zur Überdeckung der beiden letzten Strukturen (2D-Röntgenkonturen und 3D-Kernspinoberfläche, jeweils bei der Spongiosa) benutzt werden. So kann die tatsächliche Knochenoberfläche (mit Kortikalisschicht) visualisiert werden und als Grundlage für die Navigation fungieren.

Auch kann daran gedacht werden, in den in Fig. 3 dargestellten Aufnahmen 50, 50' die Kontur der Spongiosa zu markieren und nach dem erfindungsgemäßen Verfahren die SpongiosaStruktur zu visualisieren. Auf diese Weise kann die genaue räumliche Lage der Spongiosastruktur festgestellt werden. Anschließend kann die Form der Spongiosastruktur unter Verwendung des MR-Datensatzes überarbeitet werden.

In Fig. 4 ist ein zweidimensionaler Schnitt durch die in Fig. 3 dargestellte, räumliche Anordnung abgebildet. Die in Fig. 4 dargestellte Schnittebene enthält die beiden Kegelspitzen 46, 46' sowie zwei Geraden 54, 56, welche die Kegelfläche 40 begrenzen, und zwei Geraden 54', 56', welche die Kegelfläche 40' begrenzen. Der Schnittbereich dieser vier Begrenzungsgeraden 54, 56, 54', 56' legt den durch die in Fig. 4 dargestellte Ebene verlaufenden Querschnitt des Näherungsmodells 52 fest.

In Fig. 4 ist ebenfalls der tatsächliche, kreisförmige Querschnitt des Femurknochens 12 eingezeichnet. Folglich lässt sich Fig. 4 die Abweichung des Querschnitts des Näherungsmodells 52 vom tatsächlichen Querschnitt des Femurknochens 12 entnehmen. Wie aus Fig. 4 ersichtlich ist, ist die Abweichung des Näherungsmodells 52 von der tatsächlichen Gestalt des Femurknochens 12 dort besonders stark, wo sich die Begrenzungsgeraden 54, 56, 54', 56' schneiden. Diese Erkenntnis kann dazu benutzt werden, die Aufnahmepositionen im Hinblick auf die Position des geplanten, operativen Eingriffes zu optimieren.

Fig. 5 zeigt schematisch eine Möglichkeit einer intraoperativen graphischen Darstellung des nach dem vorstehend beschriebenen Visualisierungsverfahren ermittelten Näherungsmodells 52 auf dem in Fig. 1 dargestellten Bildschirm 24. Außer dem Näherungsmodell 52 wird auch die aktuelle Position des chirurgischen Instruments 14 relativ zum Näherungsmodell 52 graphisch dargestellt. Die aktuelle Position des chirurgischen Instruments 14 wird wie vorstehend erläutert mit Hilfe des in Fig. 1 dargestellten Infrarot-Tracking-Systems ermittelt. Zur Visualisierung der Form des chirurgischen Instruments 14 wird auf einen vorher abgespeicherten CAD-Datensatz zurückgegriffen.

Auf dem Bildschirm 24 werden außer dem Näherungsmodell 52 und dem chirurgischen Instrument 14 noch drei zweidimensionale Aufnahmen 50, 50', 50" des Femurknochens 12 dargestellt. Diese Aufnahmen 50, 50', 50" wurden mit Hilfe des in Fig. 1 dargestellten Röntgen-Aufnahmesystems 26 erstellt. Sie sind auf dem Bildschirm 24 bezüglich der Näherungsmodells 52 in derjenigen räumlichen Lage dargestellt, in welcher sich der Röntgendetektor 32 bei Anfertigen der jeweiligen Aufnahme 50, 50', 50" relativ zum Femurknochen 12 befand. Dies erleichtert dem Chirurgen das Navigieren des chirurgischen Instruments 14. In den Aufnahmen 50, 50', 50" ist weiterhin die jeweilige Projektion 60, 60', 60" der Wirkachse des chirurgischen Instruments 14 dargestellt.

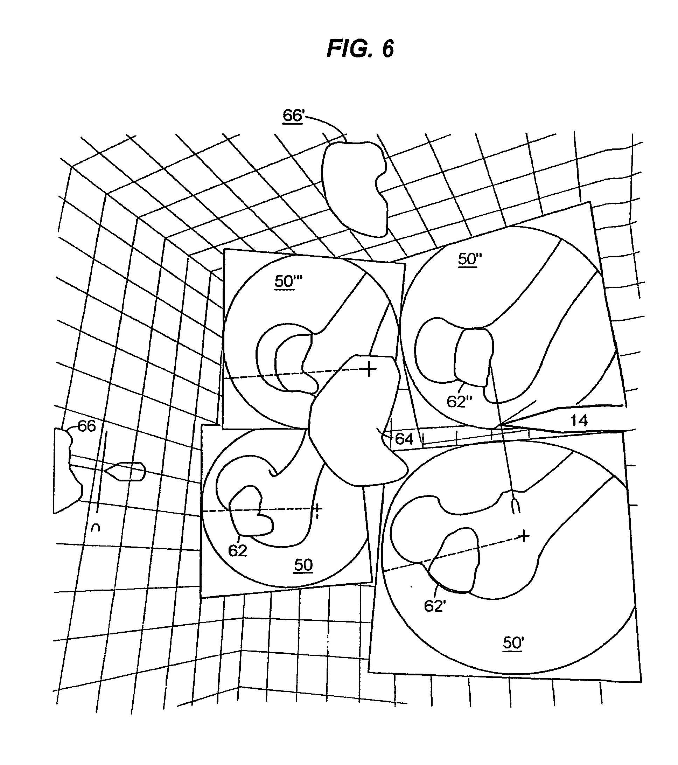

In Fig. 6 ist eine weitere Möglichkeit zur intraoperativen graphischen Darstellung des Verlaufs einer Operation in Gestalt eines Bildschirmausdrucks dargestellt. Die Darstellung gemäß Fig. 6 entspricht im wesentlichen der Darstellung gemäß Fig. 5. Abweichend von Fig. 5 ist in Fig. 6 jedoch kein dreidimensionales Näherungsmodell des gesamten Femurknochens, sondern lediglich ein dreidimensionales Näherungsmodell 64 eines Tumors, welcher den Femurknochen befallen hat, dargestellt. Das dreidimensionale Näherungsmodell 64 des Tumors basiert auf vier zweidimensionalen Aufnahmen 50, 50', 50'', 50"' des Femurknochens. Die vier Aufnahmen 50, 50', 50'', 50''' sind derart graphisch angeordnet, dass sie die jeweilige Lage des Röntgendetektors 32 relativ zum Femurknochen 12 gemäß Fig. 1 wiedergeben, in welcher die jeweilige Aufnahme 50, 50', 50", 50''' erstellt wurden.

Jede der vier Aufnahmen 50, 50', 50", 50"' enthält eine graphische Darstellung 62, 62', 62", 62''' des Tumors. Basierend auf den Umrissen der vier Darstellungen 62, 62', 62", 62"' wurden, wie unter Bezugnahme auf Fig. 3 erläutert, vier Kegelflächen generiert, deren räumlicher Schnitt dem dreidimensionalen Näherungsmodell 64 des Tumors entspricht.

In der Bildschirmdarstellung gemäß Fig. 6 sind weiterhin zwei Projektionen 66, 66' des Näherungsmodells 64 dargestellt, welche dem Chirurgen das Überwachen des chirurgischen Eingriffs zusätzlich erleichtern. Weiterhin ist in Fig. 6 die räumliche Lage des chirurgischen Instruments 14 relativ zum Näherungsmodell 64 skizziert. In jede der vier Aufnahmen 50, 50', 50", 50"' ist die entsprechende Projektion der Wirkachse 60, 60', 60" , 60"' des chirurgischen Instruments 14 skizziert. Auch diese graphische Maßnahme hilft dem Chirurgen, den operativen Eingriff intraoperativ zu überwachen.

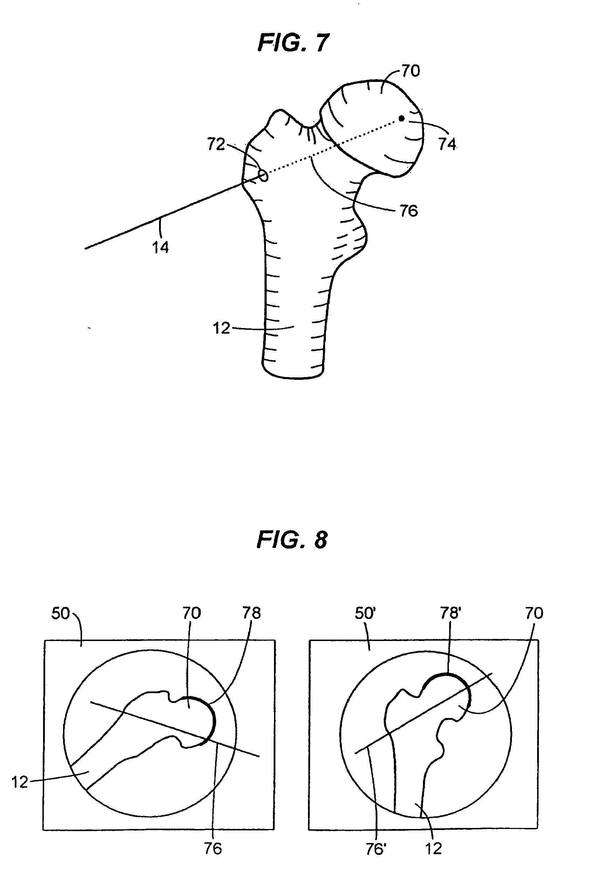

Während in Fig. 6 die Entfernung eines Tumors skizziert ist, wird nachfolgend unter Bezugnahme auf Fig. 7 der chirurgische Eingriff einer Bohrung in den kugelförmigen Kopf 70 des Femurknochens 12 anhand einer geometrischen Navigationsstruktur beschrieben. Bei einer derartigen Bohrung besteht das Problem, dass das chirurgische Instrument, nämlich der in Fig. 7 abgebildete Bohrer 14, nur bis zu einer bestimmten Eindringtiefe in den Femurknochen 12 eingeführt werden soll, da der Bohrer 14 eine Knorpelschicht auf der Oberfläche des kugelförmigen Kopfes 70 des Femurknochens 12 nicht durchstoßen darf. Der Bohrer 14, welcher an der Position 72 in den Femurknochen 12 eingeführt werden soll, darf folglich nicht so weit in den Femurknochen 12 eindringen, dass er den Durchstoßpunkt 74 erreicht.

Obwohl die Knorpelschicht auf dem kugelförmigen Kopf 70 des Femurknochens 12 in Röntgenaufnahmen 50, 50' gemäß Figur 8 sichtbar ist, kann allein aus einzelnen zweidimensionalen Röntgenaufnahmen nicht entschieden werden, ob ein Bohrer die Knorpelschicht bereits durchstoßen hat. Dies liegt daran, dass eine Röntgenaufnahme nur eine Projektion des Kopfes 70 des Femurknochens 12 liefert, welche im allgemeinen den kreisförmigen Rand des Kopfes 70 größer erscheinen lässt als er tatsächlich ist.

Das erfindungsgemäße Verfahren ermöglicht nun selbst bei Vorliegen von nur zweidimensionalen Röntgenaufnahmen 50, 50' des Femurknochens 12 ein sicheres Navigieren des Bohrers 14 derart, dass der Kopf 70, 70' des Femurknochens 12 nicht durchstoßen wird. Anhand der beiden in Fig. 8 dargestellten Aufnahmen 50, 50' wird zunächst einerseits jeweils eine Vorzugsrichtung 76, 76' gekennzeichnet, in welcher der Bohrer 14 in den Kopf 70 des Femurknochens 12 eindringen soll. Andererseits wird manuell oder über eine Software zur Konturextrahierung jeweils der halbkreisartige Umriss 78, 78' des Kopfes 70, 70' markiert. Auf diese Weise werden die Projektionen der zu visualisierenden geometrischen Strukturen festgelegt.

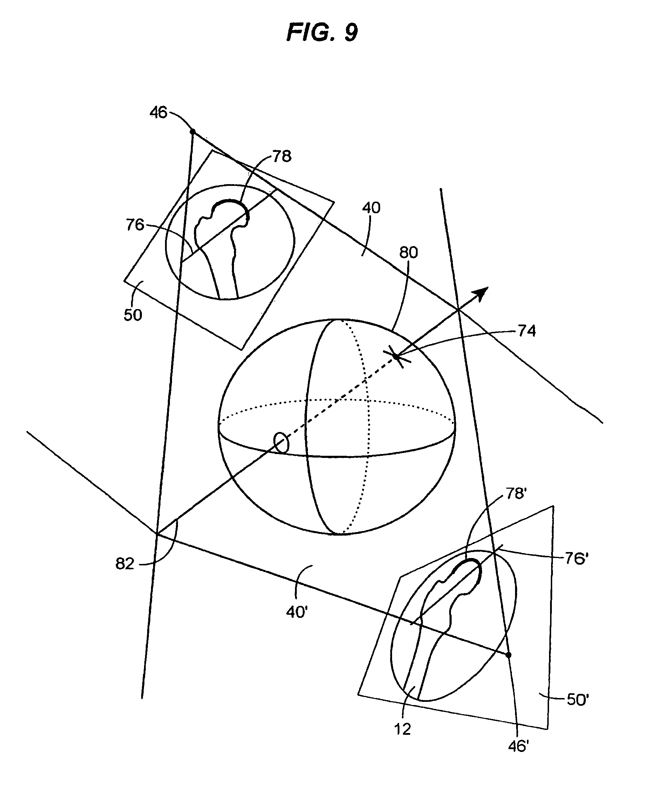

In Fig. 9 ist dargestellt, wie anhand der beiden Aufnahmen 50, 50' erfindungsgemäß die Vorzugsrichtung 76, 76' sowie eine der Oberfläche des Kopfes 70 des Femurknochens 12 entsprechende Kugel dreidimensional visualisiert werden kann. Diese Visualisierung läuft entsprechend ab wie unter Bezugnahme auf Fig. 3 und 4 vorstehend beschrieben.

Zunächst wird aus den beiden Aufnahmen 50, 50' anhand der jeweils eingezeichneten Vorzugsrichtung 76, 76' eine räumliche Gerade 82, welche dieser Vorzugsrichtung entspricht, ermittelt. Da es sich bei der in den Aufnahmen 50, 50' markierten Vorzugsrichtung 76, 76' jeweils um ein Geradenstück handelt, liegen die beiden korrespondierenden Kegelflächen 40, 40' in Gestalt eines ebenen Dreiecks vor. Wie aus Fig. 9 ersichtlich ist, ergibt sich die räumliche Gerade 82 aus dem räumlichen Schnitt der beiden durch die Kegelflächen 40, 40' definierten Ebenen. Durch den Schnitt der beiden dreiecksförmigen Flächen ist die Gerade 82, d.h. die Vorzugsrichtung eindeutig und exakt festgelegt.

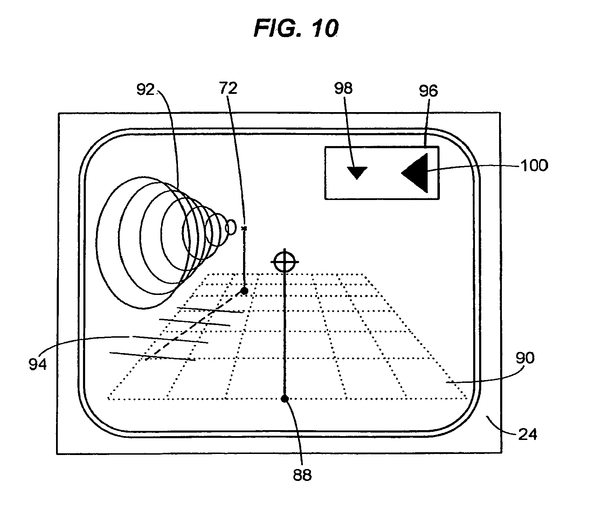

Wie vorstehend ausgeführt, kann die räumliche Gerade 82 intraoperativ zur Navigation des Bohrers 14 verwendet werden. Dieser Vorgang wird weiter unten unter Bezugnahme auf die Fig. 10 bis 12 näher beschrieben.

Zunächst wird jedoch nochmals unter Bezugnahme auf Fig. 8 und 9 erläutert, wie die räumliche Anordnung des Durchstoßpunktes 74 bezüglich der räumlichen Gerade 82 visualisiert werden kann. Zu diesem Zweck wird der räumliche Schnitt derjenigen Kegelflächen ermittelt, welche jeweils dem halbkreisförmigen Umriss 78, 78' des Kopfes 70 des Femurknochens 12 auf den Aufnahmen 50, 50' entsprechen. Wie sich aus Fig. 9 ergibt, ist der räumliche Schnitt der den halbkreisförmigen Umrissen 78, 78' entsprechenden Kegelflächen eine visualisierte Struktur in Gestalt einer räumlichen Kugel 80. Es ist darauf hinzuweisen, dass die Lage und Form der Kugel 80 anhand der Halbkreise (bzw. der entsprechenden Vollkreise) 78, 78' eindeutig und exakt festgelegt werden können. Ein Schnitt der Kugel 80 mit der räumlichen Gerade 82 ergibt die räumliche Position des Durchstoßpunktes 74.

Durch eine intraoperative Visualisierung der aktuellen Lage des Bohrers 14 relativ zum Durchstoßpunkt 74 ist es daher möglich, den Bohrer 14 derart zu navigieren, dass rechtzeitig vor dem Erreichen des Durchstoßpunktes 74 der Bohrvorgang unterbrochen werden kann. Auch die Visualisierung der räumlichen Gerade 82 gestattet ein sicheres Navigieren des Bohrers 14. Bei einer intraoperativen graphischen Darstellung von Kugel 80, Gerade 82 und Bohrer 14 wird gleichzeitig der Abstand zwischen der Spitze des Bohrers 14 und dem Durchstoßpunkt 74 z.B. in Zahlenform angezeigt. Bei Unterschreitung eines Mindestabstands ertönt ein zusätzliches akustisches Warnsignal.

Selbstverständlich können die räumliche Gerade 82 und die Kugel 80 gemäß Fig. 9 auch in den dreidimensionalen graphischen Darstellungen gemäß Fig. 5 und 6 zur Erleichterung der Navigation des chirurgischen Elements 14 dargestellt werden.

Nachfolgend wird unter Bezugnahme auf die Fig. 10 bis 12 eine erfindungsgemäße graphische Navigationshilfe für ein chirurgisches Instrument beispielhaft für den in den Fig. 7 bis 9 dargestellten operativen Eingriff erläutert. Zur Unterstützung der Navigation des in Fig. 7 dargestellten Bohrers 14 derart, dass er entlang der in Fig. 9 dargestellte räumliche Gerade 82 in den Kopf 70 des Femurknochens 12 eintritt, wird auf dem in Fig. 1 dargestellten Bildschirm 24 eine graphische Navigationshilfe dargestellt. Diese Navigationshilfe umfasst ein Fadenkreuz 86, welches die Spitze des in Fig. 7 dargestellten Bohrers 14 markiert. Der Punkt 88 bezeichnet eine Projektion der Spitze des Bohrers 14 auf eine Horizontalebene 90. Der Punkt 72 auf dem Bildschirm 24 bezeichnet den gewünschten Eintrittspunkt des Bohrers 14 in den Femurknochen 12. Die in den Fig. 10 bis 12 dargestellte Visualisierung des Punktes 72 erfolgt auf die gleiche Weise wie vorstehend unter Bezugnahme auf die Fig. 7 bis 9 für die Gerade 82 und die Kugel 80 beschrieben. Eine tunnelartige Struktur 92 dient als Anhaltspunkt für die relative Lage der räumlichen Gerade 82 relativ zur Wirkachse des Bohrers 14. Diese Wirkachse erstreckt sich in rechtem Winkel von der Position des Fadenkreuzes 86 aus der Zeichenebene heraus. Auf der Horizontalebene 90 ist weiterhin eine Projektion 94 der geometrischen Gerade 82 erkennbar.

Auf dem Bildschirm 24 rechts oben ist eine Richtungsanzeige 96 dargestellt. Diese Richtungsanzeige 96 gibt an, in welche Richtung die Spitze des Bohrers 14 zu bewegen ist, um den Bohrer 14 entlang der durch den Tunnel 92 versinnbildlichten geometrischen Geraden 82 auszurichten. In dem in der Fig. 10 dargestellten Fall muss der Bohrer 14 etwas nach unten und relativ weit nach links beweg werden. Dies ergibt sich aus einem kleinen, nach unten weisenden Pfeil im Anzeigefeld 98 und einem großen, nach links weisenden Pfeil im Anzeigefeld 100.

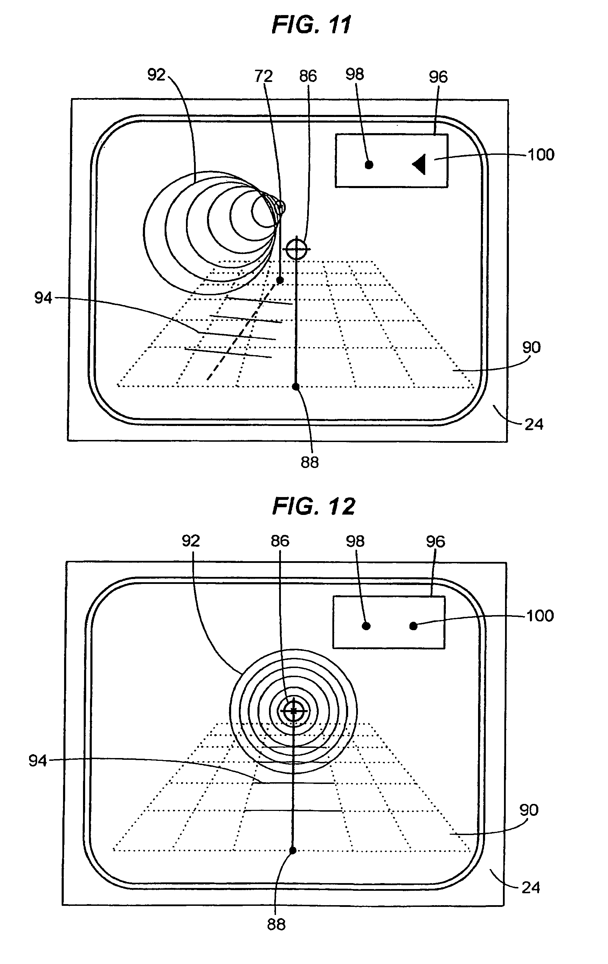

In Fig. 11 ist der Fall dargestellt, dass der Bohrers 14 ausgehend von der in Fig. 10 dargestellten Position etwas nach unten und geringfügig nach links bewegt wurde. Wie durch den Punkt im Anzeigefeld 98 angedeutet, befindet sich die durch das Fadenkreuz 86 versinnbildlichte Spitze des Bohrers 14 nun in der gleichen Horizontalebene wie die räumliche Gerade 82, welche der Tunnelstruktur 92 entspricht. Wie jedoch durch den kleinen, nach links weisenden Pfeil im Anzeigefeld 100 verdeutlicht wird, muss der Bohrer 14 noch geringfügig nach links bewegt werden, um die Wirkachse des Bohrers 14 entlang der durch den Tunnel 92 versinnbildlichten geometrischen Geraden 82 auszurichten.

Wird der Bohrer 14 nun noch geringfügig nach links bewegt, so ergibt sich der in Fig. 12 dargestellte Bildschirminhalt. In der Richtungsanzeige 96 sind nunmehr zwei Punkte dargestellt. Diese Darstellung zeigt an, dass die Wirkachse des Bohrers 14 entlang der durch die Tunnelstruktur 92 angegebenen Vorzugsrichtung ausgerichtet ist.

In dieser Position kann der Chirurg nunmehr den Bildschirm 24 in einen anderen Darstellungsmodus schalten, welcher beispielsweise entsprechend dem Bildschirmausdruck gemäß Fig. 6 eine dreidimensionale Visualisierung einer operativ zu behandelnden Struktur zu wählen.

## Patentansprüche

1. Präoperatives Verfahren zum rechnergestützten Erzeugen einer graphischen Navigationshilfe anhand dreidimensional visualisierter Strukturen, enthaltend die Schritte:
- Bereitstellen von mindestens zwei zweidimensionalen Aufnahmen (50, 50') desselben anatomischen Objekts (12) aus unterschiedlichen Aufnahmepositionen sowie von Informationen, welche einen Rückschluss auf die jeweilige räumliche Lage eines Aufnahmesystems (26) relativ zum anatomischen Objekt (12) gestatten;
- Bereitstellen digitaler Daten betreffend eine festgelegte Projektion (76, 76', 78, 78') einer zu visualisierenden geometrischen Struktur (72, 80, 82) oder eines Teils hiervon in jeder zweidimensionalen Aufnahme, wobei die zu visualisierende geometrische Struktur (72, 80, 82) vom anatomischen Objekt (12) verschieden und aus zwei Aufnahmen (50, 50') eindeutig ermittelbar ist;
- Generieren einer Kegelfläche (40, 40') im Raum für jede Aufnahme (50, 50'), wobei die räumlichen Lagen von Kegelspitze (46, 46') und Kegelleitkurve aus der jeweiligen räumlichen Lage des Aufnahmesystems (26) und die Form der Kegelleitkurve aus der Form der Projektion (76, 76', 78, 78') der zu visualisierenden geometrischen Struktur (72, 80, 82) ermittelt werden;
- Bilden eines räumlichen Schnittes der einzelnen Kegelflächen (40, 40') zur Ermittlung der geometrischen Struktur (72, 80, 82); und
- Darstellen der ermittelten geometrischen Struktur (72, 80, 82) und/oder eines Schnittes (74) mehrerer ermittelter geometrischer Strukturen (72, 80, 82) als Navigationshilfe.

2. Verfahren nach Anspruch 1, **dadurch gekennzeichnet, dass** die festgelegte Projektion der geometrischen Struktur (72, 80, 82) ein Punkt, eine Gerade (76, 76'), ein Kreissegment (78, 78') oder eine andere linienartige Struktur ist.

3. Verfahren nach Anspruch 1 oder 2, **dadurch gekennzeichnet, dass** die zu visualisierende geometrische Struktur ein Punkt (72), eine Gerade (82), eine Ebene, eine Kugel (80) oder eine andere zwei- oder dreidimensionale Struktur ist.

4. Verfahren nach Anspruch 1, 2 oder 3, **dadurch gekennzeichnet, dass** die zweidimensionalen Aufnahmen (50, 50') durch Röntgenverfahren und/oder Magnetoresonanzverfahren generiert werden.

5. Verfahren nach einem der Ansprüche 1 bis 4, **dadurch gekennzeichnet, dass** der räumliche Schnitt anhand mindestens eines weiteren Datensatzes des anatomischen Objekts (12) überarbeitet wird.

6. Verfahren nach Anspruch 5, **dadurch gekennzeichnet, dass** als weiterer Datensatz eine zweidimensionale oder dreidimensionale Aufnahme oder ein generisches Modell des anatomischen Objekts (12) verwendet wird.

7. Verfahren nach einem der Ansprüche 1 bis 6, **dadurch gekennzeichnet, dass** durch inverse Berechnungen geeignete Aufnahmepositionen für weitere zwei**dimensionale Aufnahmen ermittelt werden.**

8. Verfahren nach einem der Ansprüche 1 bis 7, **dadurch gekennzeichnet, dass** zusätzlich die räumliche Lage eines zu navigierenden Instruments (14) relativ zur geometrischen Struktur (72, 80, 82) oder zum Schnitt (74) graphisch dargestellt wird.

9. Verfahren nach einem der Ansprüche 1 bis 8, **dadurch gekennzeichnet, dass** die Wirkachse (60, 60', 60", 60"') des chirurgischen Instruments (14) graphisch dargestellt wird.

10. Verfahren nach einem der Ansprüche 1 bis 9, **dadurch gekennzeichnet, dass** die einzelnen zweidimensionalen Aufnahmen (50, 50', 50", 50"') unter Berücksichtigung ihrer jeweiligen Aufnahmeposition (46, 46') graphisch dargestellt werden.

11. Verfahren nach einem der Ansprüche 1 bis 10, **dadurch gekennzeichnet, dass** eine Navigationshilfe in Form einer Tunnelstruktur (92) graphisch dargestellt wird.

12. Verfahren nach Anspruch 11, **dadurch gekennzeichnet, dass** außer der Navigationshilfe (92) eine separate Richtungsanzeige (96) für die Navigation des chirurgischen Instruments (14) graphisch dargestellt wird.

13. Computerprogrammprodukt mit Programmcodemitteln zum Durchführen eines Verfahrens nach einem der Ansprüche 1 bis 12 wenn das Computerprogrammprodukt auf einem Rechner (22) abläuft.

14. Computerprogrammprodukt nach Anspruch 13, gespeichert auf einem Rechner (22).

15. System zum rechnergestützten Erzeugen einer graphischen Navigationshilfe anhand dreidimensional visualisierter Strukturen, enthaltend:
- Mittel (18, 20, 26) zum Bereitstellen mindestens zweier zweidimensionaler Aufnahmen (50, 50') desselben anatomischen Objekts (12) aus unterschiedlichen Aufnahmepositionen sowie von Informationen, welche einen Rückschluss auf die jeweilige räumliche Lage eines Aufnahmesystems (26) relativ zum anatomischen Objekt (12) gestatten;
- Mittel (22) zum Bereitstellen digitaler Daten betreffend eine festgelegte Projektion (76, 76', 78, 78') einer zu visualisierenden geometrischen Struktur (72, 80, 82) oder eines Teils hiervon in jeder zweidimensionalen Aufnahme, wobei die zu visualisierende geometrische Struktur (72, 80, 82) vom anatomischen Objekt (12) verschieden und aus zwei Aufnahmen (50, 50') eindeutig ermittelbar ist, zum Generieren einer Kegelfläche (40, 40') im Raum für jede Aufnahme (50, 50'), wobei die räumlichen Lagen von Kegelspitze (46, 46') und Kegelleitkurve aus der jeweiligen räumlichen Lage des Aufnahmesystems (26) und die Form der Kegelleitkurve aus der Form der Projektion (76, 76', 78, 78') der zu visualisierenden geometrischen Struktur (72, 80, 82) ermittelt werden, und zum Bilden eines räumlichen Schnittes der einzelnen Kegelflächen (40, 40') zur Ermittelung der geometrischen Struktur (72, 80, 82); und
- Mittel (24) zum Darstellen der ermittelten geometrischen Struktur (72, 80, 82) und/oder eines Schnittes (74) mehrerer ermittelter geometrischer Strukturen (72, 80, 82) als Navigationshilfe.

## Claims

1. A preoperative method for the computer-supported generation of a graphic navigating aid by means of three-dimensionally visualized structures, comprising the steps of:
- providing at least two two-dimensional images (50, 50') of the same anatomical object (12) from different perspectives and also of information which makes it possible to draw a conclusion about the respective spatial position of an imaging system (26) relative to the anatomical object (12);
- providing digital data relating to a defined projection (76, 76', 78, 78') of a geometrical structure (72, 80, 82) to be visualized or a part thereof in each two-dimensional image, wherein the geometrical structure (72, 80, 82) to be visualized is different from the anatomical object (12) and can uniquely be determined from two images (50, 50');
- generating a conical surface (40, 40') in space for every image (50, 50'), wherein the spatial positions of cone vertex (46, 46') and cone directrix are determined from the respective spatial position of the imaging system (26) and the shape of the cone directrix is determined from the shape of the projection (76, 76', 78, 78') of the geometrical structure (72, 80, 82) to be visualized;
- forming a spatial intersection of the individual conical surfaces (40, 40') to determine the geometrical structure (72, 80, 82); and
- displaying the determined geometrical structure (72, 80, 82) and/or an intersection (74) of several geometrical structures (72, 80, 82) determined as navigation aid.

2. Method according to claim 1, **characterized in that** the defined projection of the geometrical structure (72, 80, 82) is a point, a straight line (76, 76'), a circular segment (78, 78') or another line-type structure.

3. Method according to claim 1 or 2, **characterized in that** the geometrical structure to be visualized is a point (72), a straight line (82), a plane, a sphere (80) or another two-dimensional or three-dimensional structure.

4. Method according to Claim 1, 2 or 3, **characterized in that** the two-dimensional images (50, 50') are generated by X-ray methods and/or magnetoresonance methods.

5. Method according to one of Claims 1 to 4, **characterized in that** the spatial intersection is revised using at least one further data set of the anatomical object (12).

6. Method according to Claim 5, **characterized in that** a two-dimensional or three-dimensional image or a generic model of the anatomical object (12) is used as further data set.

7. Method according to one of Claims 1 to 6, **characterized in that** suitable perspectives are determined for further two-dimensional images by inverse calculations.

8. Method according to one of Claims 1 to 7, **characterized in that**, additionally, the spatial position of an instrument (14) to be navigated is shown graphically relative to the geometrical structure (72, 80, 82) or to the intersection (74).

9. Method according to one of Claims 1 to 8, **characterized in that** the effective axis (60, 60', 60", 60"') of the surgical instrument (14) is shown graphically.

10. Method according to one of Claims 1 to 9, **characterized in that** the individual two-dimensional images (50, 50', 50", 50"') are shown graphically taking account of the positions (46, 46') from which the images were taken in each case.

11. Method according to one of Claims 1 to 10, **characterized in that** a navigation aid in the form of a tunnel structure (92) is shown graphically.

12. Method according to Claim 11, **characterized in that**, in addition to the navigation aid (92), a separate direction indicator (96) for navigating the surgical instrument (14) is shown graphically.

13. A computer program product with program code means for carrying out a method according to one of claims 1 to 12 when the computer program is running on a computer (22).

14. Computer program product according to claim 13, stored in a computer.

15. A system for the computer-supported generation of a graphic navigation aid by means of three-dimensionally visualized structures, comprising:
- means (18, 20, 26) for providing at least two two-dimensional images (50, 50') of the same anatomic object (12) from different perspectives and of information that makes it possible to draw conclusions about the respective spatial position of an imaging system (26) relative to the anatomic object (12);
- means (22) for providing digital data concerning a defined projection (76, 76', 78, 78') of a geometrical structure (72, 80, 82)to be visualized or of a part thereof in every two-dimensional image, wherein the geometrical structure (72, 80, 82) to be visualized is different from the anatomic object (12) and can uniquely be determined from two images (50, 50'), for generating a conical surface (40, 40') in space for every image (50, 50'), wherein the spatial positions of cone vertex (46, 46') and cone directrix are determined from the respective spatial position of the imaging system (26) and the shape of the cone directrix is determined from the shape of the projection (76, 76', 78, 78') of the geometrical structure (72, 80, 82) to be visualized, and for forming a spatial intersection of the individual cone surfaces (40, 40') to determine the geometrical structure (72, 80, 82); and
- means (24) for depicting the determined geometrical structure (72, 80, 82) and/or an intersection (74) of several geometrical structures (72, 80, 82) determined as navigation aid.

## Revendications

1. Procédé préopératoire permettant la conception assistée par ordinateur d'un outil graphique d'aide à la navigation au moyen de structures visualisées en trois dimensions, comprenant les étapes suivantes :
- la mise à disposition d'au moins deux images bidimensionnelles (50, 50') du même objet anatomique (12) prises sous différents angles, ainsi que d'informations permettant de déterminer par déduction la position spatiale respective d'un système de prise de vue (26) par rapport à l'objet anatomique (12) ;
- la mise à disposition de données numériques concernant une projection déterminée (76, 76', 78, 78') dans chaque image bidimensionnelle d'une structure (72, 80, 82) géométrique à visualiser ou d'une partie de celle-ci, la structure géométrique à visualiser (72, 80, 82) étant différente de l'objet anatomique (12) et pouvant être nettement déterminée à partir de deux images (50, 50') ;
- la production dans l'espace d'une surface conique (40, 40') pour chaque image (50, 50'), les positions spatiales de l'extrémité du cône (46, 46') et de la ligne directrice du cône étant déterminées à partir de la position spatiale respective du système de prise de vue (26), et la forme de la ligne directrice du cône, à partir de la forme de la projection (76, 76', 78, 78') de la structure géométrique à visualiser (72, 80, 82) ;
- la formation d'une coupe spatiale de chaque surface conique (40, 40') pour déterminer la structure géométrique (72, 80, 82) ; et
- la représentation de la structure géométrique (72, 80, 82) ainsi déterminée et/ou d'une coupe (74) de plusieurs structures géométriques (72, 80, 82) ainsi déterminées pour servir à la navigation.

2. Procédé selon la revendication 1, **caractérisé en ce que** la projection déterminée de la structure géométrique (72, 80, 82) est un point, une droite (76, 76'), un segment de cercle (78, 78') ou une autre structure linéaire.

3. Procédé selon la revendication 1 ou 2, **caractérisé en ce que** la structure géométrique à visualiser est un point (72), une droite (82), un plan, une sphère (80) ou une autre structure bi- ou tridimensionnelle.

4. Procédé selon la revendication 1, 2 ou 3, **caractérisé en ce que** les images bidimensionnelles (50, 50') sont obtenues par procédé radiographique et/ou par procédé d'imagerie par résonance magnétique.

5. Procédé selon l'une des revendications 1 à 4, **caractérisé en ce qu'**il est utilisé au moins un autre jeu de données de l'objet anatomique (12) pour le traitement de ladite coupe spatiale.

6. Procédé selon la revendication 5, **caractérisé en ce que** l'autre jeu de données utilisé est une image bidimensionnelle ou tridimensionnelle ou un modèle généré à partir de l'objet anatomique (12).

7. Procédé selon l'une des revendications 1 à 6, **caractérisé en ce que** des angles appropriés pour la prise d'autres images bidimensionnelles sont déterminés par calculs inverses.

8. Procédé selon l'une des revendications 1 à 7, **caractérisé en ce que** la position dans l'espace d'un instrument à naviguer (14) est en outre représentée graphiquement par rapport à la structure géométrique (72, 80, 82) ou à la coupe (74).

9. Procédé selon l'une des revendications 1 à 8, **caractérisé en ce que** l'axe de travail (60, 60', 60", 60"') de l'instrument chirurgical (14) est représenté graphiquement.

10. Procédé selon l'une des revendications 1 à 9, **caractérisé en ce que** chacune des images bidimensionnelles (50, 50', 50", 50"') est représentée graphiquement en tenant compte de l'angle (46, 46') sous lequel chacune a été prise.

11. Procédé selon l'une des revendications 1 à 10, **caractérisé en ce qu'**un outil d'aide à la navigation est représenté graphiquement sous la forme d'une structure tunnellaire (92).

12. Procédé selon la revendication 11, **caractérisé en ce que**, outre l'outil d'aide à la navigation (92), il est représenté graphiquement un indicateur directionnel (96) pour la navigation de l'instrument chirurgical (14).

13. Programme informatique comprenant des moyens de codage pour la mise en oeuvre d'un procédé selon l'une des revendications 1 à 12 lorsque le programme informatique tourne sur un ordinateur (22).

14. Programme informatique selon la revendication 13, enregistré sur un ordinateur (22).

15. Système permettant la conception assistée par ordinateur d'un outil graphique d'aide à la navigation au moyen de structures visualisées en trois dimensions, comprenant les étapes suivantes :
- des moyens (18, 20, 26) permettant la mise à disposition d'au moins deux images bidimensionnelles (50, 50') du même objet anatomique (12) prises sous différents angles, ainsi que d'informations permettant de déterminer par déduction la position spatiale respective d'un système de prise de vue (26) par rapport à l'objet anatomique (12) ;
- des moyens (22) permettant la mise à disposition de données numériques concernant une projection déterminée (76, 76', 78, 78') dans chaque image bidimensionnelle d'une structure (72, 80, 82) géométrique à visualiser ou d'une partie de celle-ci, la structure géométrique à visualiser (72, 80, 82) étant différente de l'objet anatomique (12) et pouvant être nettement déterminée à partir de deux images (50, 50'), permettant la production d'une surface conique (40, 40') pour chaque image (50, 50'), les positions spatiales de l'extrémité du cône (46, 46') et de la ligne directrice du cône étant déterminées à partir de la position spatiale respective du système de prise de vue (26), et la forme de la ligne directrice du cône, à partir de la forme de la projection (76, 76', 78, 78') de la structure géométrique à visualiser (72, 80, 82), ainsi que la formation d'une coupe spatiale de chaque surface conique (40, 40') pour déterminer la structure géométrique (72, 80, 82) ; et
- des moyens (24) permettant la représentation de la structure géométrique (72, 80, 82) ainsi déterminée et/ou d'une coupe (74) de plusieurs structures géométriques (72, 80, 82) ainsi déterminées pour servir à la navigation.
